(19) Europäisches Patentamt
European Patent Office
Office européen des brevets

(11) Numéro de publication: 0 184 572
B1

(12) # FASCICULE DE BREVET EUROPÉEN

(45) Date de publication du fascicule du brevet: **17.01.90**

(51) Int. Cl.[4]: **C 07 C 59/48**, C 07 C 51/00, C 07 D 333/38

(21) Numéro de dépôt: **85870165.9**

(22) Date de dépôt: **28.11.85**

(54) **Procédé de préparation d'acides alpha-hydroxy-alcanoiques.**

(30) Priorité: **29.11.84 FR 8418201**

(43) Date de publication de la demande: **11.06.86 Bulletin 86/24**

(45) Mention de la délivrance du brevet: **17.01.90 Bulletin 90/03**

(84) Etats contractants désignés: **AT BE CH DE FR GB IT LI LU NL SE**

(56) Documents cité:
**EP-A-0 071 299**

**J.A.C.S. 72, 1642-1644 (1950)**

(73) Titulaire: **SANOFI S.A.**
**40, Avenue George V**
**F-75008 Paris (FR)**
Titulaire: **INDUSTRIA CHIMICA PRODUTTI FRANCIS S.p.A.**
**Via Origgio, 23**
**I-21042 Caronno P. LLa. (VA) (IT)**

(72) Inventeur: **Andre, Jean-Daniel**
**Square Horizon Bâtiment B**
**F-04200 Sisteron (FR)**
Inventeur: **Heymes, Alain**
**Rue Frédéric Mistral, 5**
**F-04200 Sisteron (FR)**
Inventeur: **Grossi, Pierre-Jean**
**Rue des Chapelles, 2**
**F-30390 Aramon (FR)**
Inventeur: **Venerucci Manzarolli, Dott. Giovanni**
**Via Guerrazzi, 25**
**I-20145 Milano (IT)**

(74) Mandataire: **Bressand, Georges**
**c/o CABINET LAVOIX 2 Place d'Estienne d'Orves**
**F-75441 Paris Cédex 09 (FR)**

EP 0 184 572 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dípposition (Art. 99(1) Convention sur le brevet européen).

LIBERGRAF, STOCKHOLM 1990

## Description

La présente invention se rapporte, d'une manière générale, à un nouveau procédé de préparation d'acides α-hydroxy-alcanoiques.

L'invention concerne notamment un nouveau procédé de préparation d'acides α-hydroxy-alcanoiques de formule générale:

```
   OH
   |
Cy–C–CO2H          I
   |
   R
```

dans laquelle R représente hydrogène ou un radical alkyle inférieur et Cy représente un radical phényle ou hétérocyclique, ce radical comportant éventuellement un ou plusieurs substituants sélectionnés du groupe formé par des radicaux alkyle inférieur, alkényle inférieur, alkynyle inférieur et par des atomes d'halogène tels que chlore ou brome.

Dans le présent contexte, les termes repris ci-dessus comportent la signification suivante:

"alkyle inférieur" désigne les restes d'hydrocarbures aliphatiques saturés ayant jusqu'à 4 atomes de carbone tels que méthyle, éthyle, n-propyle, isopropyle, n-butyle, isobutyle ou tertiobutyle;
"alkényle inférieur" désigne les restes d'hydrocarbures aliphatiques insaturés comportant une ou deux doubles liaisons et ayant de 2 à 4 atomes de carbone tels que vinyle, allyle ou butènyle;
"alkynyle inférieur" désigne les restes d'hydrocarbures aliphatiques insaturés comportant une ou deux triples liaisons et ayant de 2 à 4 atomes de carbone tels qu'éthynyle, propargyle ou butynyle;
"radical hétérocyclique" désigne essentiellement un radical furyle, thiényle, pyrrolyle, benzofuryle, benzothiényle ou indolyle.

Ainsi, en tenant compte des valeurs données ci-dessus, le radical Cy peut représenter notamment un radical isobutylphényle, de préférence isobutyl-4 phényle ou un radical chloro-thiényle, de préférence chloro-5 thiényl-2.

Ces composés sont particulièrement utiles notamment comme intermédiaires dans la synthèse des acides alcanoiques de formule générale:

```
       O
       ||
Cy–CH–COH          (I')
   |
   R
```

dans laquelle R et Cy ont la même signification que précédemment.

Parmi ces composés qui sont connus notamment comme agents anti-inflammatoires, antipyrétiques ou antalgiques, on peut citer l'acide (isobutyl-4 phényl)-2 acétique ou ibufenac, l'acide (isobutyl-4 phényl)-2 propionique ou ibuprofène ou l'acide (isobutyl-4 phényl)-2 butyrique ou butibufène.

On a décrit dans la demande de brevet japonais No. 54 - 39042 (C.A. *91*, P 140574 u) la synthèse de l'ibuprofène à partir d'acide (isobutyl-4 phényl)-2 hydroxy-2 propionique ainsi que la préparation de cet intermédiaire.

Suivant le procédé cité, on prépare l'α-hydroxy-acide en question à partir d'(isobutyl-4 phényl)-2 oxo-2 acétate d'éthyle par réaction avec un halogénure de méthyl magnésium en milieu éther suivie d'une hydrolyse alcaline selon les conditions de la réaction de GRIGNARD.

Bien que les rendements obtenus soient importants, les conditions opératoires d'une telle réaction sont couteûses et difficiles à mettre en oeuvre sur le plan industriel (utilisation du magnésium, milieu réactionnel anhydre etc...).

D'autres publications rapportent également des procédés de préparation des acides α-hydroxy-alcanoiques de formule (I').

Dans la plupart des cas, ces procédés comportent des inconvénients les écartant d'une mise en oeuvre industrielle, inconvénients qui peuvent provenir par exemple de l'utilisation de produits de départ relativement difficiles à obtenir.

On connaît cependant un procédé de préparation de certains acides αhydroxy-phénylalcanoiques faisant intervenir des conditions opératoires extrapolables sans difficultés majeures sur le plan industriel.

Ce procédé, qui a été décrit notamment dans J.A.C.S. *72*, 1642 - 1644 (1950) ou dans Org. Synth. III, 538 - 541 (1955) repose sur une réaction de transposition de phénylalkylcétones α,α-dihalogénées faisant intervenir l'hydroxyde de sodium aqueux.

On a tenté de préparer les acides de formule I et en particulier l'acide (isobutyl-4 phényl)-2 hydroxy-2 propionique suivant une telle méthode mais avec un succès médiocre.

Les rendements maximum obtenus n'ont été que de 19 % environ en acide désiré, l'acide isobutyl-4 benzoïque étant le produit préférentiellement synthétisé. Par conséquent, l'obtention des acides α-hydroxy-phénylalca-

noïques de formule I suivant un procédé exploitable industriellement reste d'un intérêt primordial.

On a maintenant découvert, suivant la présente invention, qu'il est possible de préparer les α-hydroxy-acides de formule I, selon un tel procédé industriel, au moyen d'une réaction de transposition de cétones α,α-dihalogénées en faisant intervenir un hydroxyde de métal alcalin en solution aqueuse et un solvant organique non polaire.

Ainsi, le procédé de l'invention pour la préparation des acides αhydroxy-alcanoïques en question consiste:

– à traiter une cétone α,α-dihalogénée de formule générale:

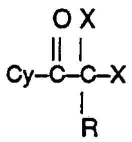

II

dans laquelle R et Cy ont la même signification que précédemment et X représente chlore, brome ou iode en présence d'une solution aqueuse d'un hydroxyde de métal alcalin tel que l'hydroxyde de lithium, de sodium ou de potassium et d'un solvant organique non polaire sélectionné d'un hydrocarbure aromatique ou alicyclique et ce, à une température comprise entre la température d'ébullition du milieu réactionnel à pression atmosphérique et 240°C sous pression,

– puis à acidifier le sel de métal alcalin ainsi formé pour obtenir l'acide désiré.

Dans le cas particulier de l'acide (isobutyl-4 phényl)-2 hydroxy-2 propionique, le procédé de l'invention permet d'obtenir des rendements de l'ordre de 70 à 90 % tout en limitant la formation de dérivés d'acide benzoïque produits par une réaction secondaire.

Comme hydrocarbure aromatique, on peut utiliser par exemple le toluène, les xylènes, l'isobutylbenzène ou le naphtalène et comme hydrocarbure alicyclique, par exemple le cyclohexane.

On préfère généralement les xylènes et en particulier le xylène industriel pour des raisons économiques.

A la pression atmosphérique et à la température d'ébullition du mélange réactionnel, la réaction de transposition s'effectuera en 20 à 25 heures avec des rendements d'au moins 70 %.

Sans être limitatives, les conditions opératoires suivantes seront généralement utilisées:

0,5 à 40 parties en poids d'hydroxyde de métal alcalin
5 à 400 parties en volume d'eau
2 à 40 parties en volume de solvant organique

et ce, pour 1 partie en poids de cétone de formule II, la réaction ayant lieu à la température d'ébullition du mélange formé.

On a trouvé, en particulier, que la réaction de transposition selon l'invention peut être considérablement accélérée par élévation de la température.

On a, en effet, remarqué, qu'une élévation de la température réactionnelle, de préférence entre 160 et 240°C nécessitant des conditions opératoires sous pression, produit le même effet qu'une augmentation de la durée de la réaction.

Ainsi, à une température de 180 à 220°C, la réaction de transposition au sein du milieu réactionnel maintenu sous agitation et sous pression, par exemple par l'utilisation d'un autoclave agité par balancement, peut être accomplie en quelques minutes par exemple en 15 à 60 minutes.

A ces températures comprises entre la température d'ébullition du milieu réactionnel à pression atmosphérique et 240°C sous pression, les conditions opératoires suivantes seront généralement utilisées sans pour autant constituer une limitation au procédé de l'invention:

0,5 à 10 parties en poids d'hydroxyde de métal alcalin
5 à 150 parties en volume d'eau
1 à 30 parties en volume de solvant organique

et ce, pour 1 partie en poids de cétone de formule II.

On a remarqué, en outre, qu'aux températures supérieures à la température d'ébullition du mélange, la réaction de transposition selon l'invention fournit les sels de métaux alcalins des α-hydroxy-acides de formule I selon des rendements particulièrement élevés.

A 160°C, 180°C, 200°C ou à 220°C, la réaction ne produit pas plus de 8 % de dérivé d'acide benzoïque et jusqu'à 90 % d'α-hydroxy-acide de formule I.

Selon un premier mode de mise en oeuvre du procédé de l'invention à une température comprise entre la température d'ébullition du milieu réactionnel à pression atmosphérique et 240°C sous pression, on mélange, dans l'autoclave, l'hydroxyde de métal alcalin en solution, le xylène et la cétone dihalogénée de formule II et on chauffe l'autoclave fermé à la température désirée.

On peut également pratiquer selon un second mode opératoire qui consiste à:

- charger au départ dans l'autoclave la solution aqueuse d'hydroxyde de métal alcalin ainsi que le xylène si la cétone dihalogénée de formule II est liquide,
- chauffer l'autoclave fermé jusqu'à l'obtention de la température désirée puis à introduire, à l'aide d'une pompe doseuse, la cétone dihalogénée de formule II telle quelle ou, en solution dans le xylène. Généralement l'introduction de la cétone dihalogénée s'effectue en 2 heures environ.

Cette dernière façon d'opérer, par addition en continu de la cétone dihalogénée permet d'accroître considérablement la productivité en α-hydroxy-acide de formule I qui peut atteindre 7 % (79/100 ml) du milieu réactionnel.

La séparation et la purification des α-hydroxy-acides de formule I peut être obtenue par précipitation de leurs sels métalliques à partir du mélange réactionnel puis acidification. Cette mise en oeuvre évite la nécessité d'acidifier la totalité du mélange contenant un excès de base et l'obligation d'extraire les α-hydroxy-acides en question au sein d'un volume d'eau important. De plus, un avantage non négligeable provient du fait qu'au cours de la précipitation des sels métalliques des α-hydroxy-acides, les autres impuretés acides de même que les halogénures de sodium formés ne sont pas entraînés dans le précipité et demeurent presque totalement en raison de leurs plus grandes solubilités aussi bien dans le milieu réactionnel que dans l'eau. Ces sels d'α-hydroxy-acides peuvent donc être éliminés par filtration. Cet avantage est d'autant plus intéressant car les réactions ultérieures conduisant aux acides alcanoïques de formule (I') ne produisent pas ou peu d'impuretés.

On pourra donc obtenir ces acides alcanoïques purs par purification aisée des produits de départ en l'occurrence les acides α-hydroxy-alcanoïques de formule I alors que selon d'autres procédés antérieurs, la purification ne peut se faire qu'au niveau de l'acide alcanoïque, purification qui se révèle difficile à ce stade.

Un autre avantage du procédé de l'invention se situe au niveau de la mise en oeuvre des composés de départ particulièrement intéressants, à savoir les composés de formule II, en raison de la facilité avec laquelle ils peuvent être produits.

Ces composés de formule II peuvent être obtenus par exemple à partir d'une cétone de formule générale:

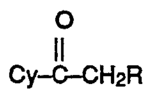

III

dans laquelle R et Cy ont la même signification que précédemment soit par chloration dans le N,N-diméthylformamide à 80 - 100°C selon la méthode décrite dans Synth. Commun., *9*, 575 - 582 (1979) soit par l'action du brome en excès dans l'acide acétique selon la méthode citée dans Organic Synthesis, IV, 110 - 113 (1963), la cétone de formule III étant elle-même obtenue au départ d'un composé de formule Cy-H dans lequel Cy a la même signification que précédemment, par réaction de Friedel-Crafts avec les chlorures d'acyle de formule générale:

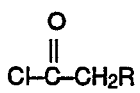

IV

dans laquelle R a la même signification que précédemment.

Selon une variante, on peut obtenir directement les composés de formule II dans laquelle Cy représente un radical phényle tel que défini dans la formule I par acylation selon les conditions de la réaction de Friedel-Crafts, au moyen des halogénures d'acyle α,α-dihalogénés de formule générale:

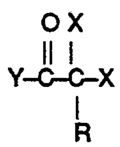

V

dans laquelle R et X ont la même signification que précédemment et Y représente chlore ou brome, ou au moyen des anhydrides ou autres dérivés équivalents des composés de formule V ci-dessus.

Comme indiqué précédemment, les acides α-hydroxy-alcanoiques de formule I peuvent être utilisés comme intermédiaires de synthèse pour la préparation des acides alcanoïques de formule (I') ci-dessus.

A cet effet, les composés de formule I pourront être mis en oeuvre suivant des procédures telles que:

a) déshydratation au reflux dans un solvant au moyen, par exemple d'acide p-toluènesulfonique suivie d'hydrogénation catalytique dans un solvant de l'acide alcène-2 oïque obtenu, par application de la méthode décrite dans la demande de brevet de la République Fédérale d'Allemagne No. 2 613 817.
b) hydrogénolyse des α-hydroxy-acides par exemple en présence de nickel de Raney dans l'acide acétique à 170°C et sous 16 atmosphères tel que décrit dans la demande de brevet japonais No. 53 - 02449 (C.A. *89*, 6118 d) ou en présence de charbon palladié dans l'acide acétique à 60°C et sous 30 atmosphères comme décrit dans la demande de brevet japonais No. 53 - 34745 (C.A., *89*, 108684 e).

Il est également possible de modifier les méthodes antérieures ci-dessus en utilisant soit l'acide sulfurique comme

catalyseur, ce qui permet d'opérer à pression atmosphérique soit l'acide iodhydrique dans l'acide acétique.

Comme mentionné précédemment, le procédé de l'invention s'est révélé de loin supérieur aux procédés suggérés par l'état de la technique où interviennent des réactions de transposition de cétones α,α-dihalogénées.

A cet effet, on a pratiqué des essais de transposition de la dichloro-2,2 (isobutyl-4 phényl)-1 propanone-1 en acide (isobutyl-4 phényl)-2 hydroxy-2 propionique suivant, d'une part le procédé de l'invention et d'autre part des conditions selon l'art antérieur.

On a obtenu les résultats suivants:

a) selon l'invention

| Réactifs et solvant pour 1 g de cétone | | | Réaction de transposition | | Rendements molaires (%) | |
|---|---|---|---|---|---|---|
| NaOH (g) | eau (ml) | Xylène (ml) | Température | Durée (h) | A* | B** |
| 20 | 400 | 40 | Ebullition | 24 | 85 | 2 |
| 20 | 200 | 40 | Ebullition | 24 | 75 | 3 |

b) selon l'art antérieur

| Conditions opératoires | Rendements molaires (%) | |
|---|---|---|
| | A* | B** |
| NaOH 20 %/eau/20°C/29 h | 0 | 7 |
| NaOH 20 %/eau/60°C/2,5 h + éthanol/1h supplémentaire | 0 | 33 |
| NaOH 32 %/eau/20°C/6 h + éthylèneglycol + éthanol/1h | 19 | 40 |

* acide (isobutyl-4 phényl)-2 hydroxy-2 propionique
** acide isobutyl-4 benzoïque

Ces résultats montrent la très nette supériorité du procédé de l'invention tant au point de vue du rendement élevé en α-hydroxy-acide formé que de la faible proportion d'acide isobutyl-4 benzoïque formé par une réaction secondaire.

Les Exemples, non limitatifs suivants, illustrent le procédé de l'invention:

**Exemple 1**

*Préparation de l'acide (isobutyl-4 phényl)-2 hydroxy-2 propionique*

a) *(Isobutyl-4 phényl)-1 propanone-1*

On ajoute en 30 minutes à 20 - 25°C, 102 g (1,1 mole) de chlorure de propionyle à une suspension de 140 g (1,05 mole) de chlorure d'aluminium dans 540 ml de chlorure de méthylène anhydre. On obtient une solution jaune pâle à laquelle on ajoute en 25 minutes à 20 - 25°C, 134 g (1 mole) d'isobutylbenzène.

On agite le milieu réactionnel pendant 90 minutes à 20 - 25°C puis on introduit, 500 ml d'acide chlorhydrique à 10 % sans dépasser 25°C en refroidissant par un bain d'eau glacée. On décante et extrait la phase aqueuse au chlorure de méthylène. On lave les phases organiques à l'eau salée, sèche sur sulfate de sodium et amène à sec sous vide. On distille sous vide le produit brut.

On obtient ainsi 179 g d'(isobutyl-4 phényl)-1 propanone-1 titrant 95,9 % en chromatographie phase gazeuse (C.P.G.) soit un rendement molaire de 90,2 %. *P.E.*: 100 - 103°C sous 0,2 mm Hg

On prépare un échantillon analytique de ce dérivé par chromatographie préparative sur plaque de silice avec comme éluant l'hexane à 4 % d'acétate d'éthyle.

*C.P.G.*: 99,7 %

**Analyse**

| | | | | | | |
|---|---|---|---|---|---|---|
| C | calculé | : | 82,06 % | trouvé | : | 81,46 % |
| H | calculé | : | 9,54 % | trouvé | : | 9,17 % |

*I.R.* (film)
1675, 1610, 780 $cm^{-1}$

*R.M.N.* ($CCl_4$)
7,80 et 7,15 (2d, 4H); 2,90 (q, 2H); 2,50 (d, 2H); 1,90 (m, 1H); 1,15 (t, 3H); 0,90 ppm (d, 6H)

$n_D^{22} = 1,5145$

En opérant comme décrit ci-dessus avec le chlorure de butyryle à la place du chlorure de propionyle, on obtient 193,5 g d'(isobutyl-4 phényl)-1 butanone-1 titrant 95,2 % en *C.P.G.* soit un rendement molaire de 90,3 %. *P.E.*: 115 - 119°C sous 1 mm Hg

On prépare un échantillon analytique de ce dérivé par distillation.

*P.E.:* 117 - 119°C sous 1 mm Hg

*C.P.G.:* 97,5 %

**Analyse**

| | | | | | | |
|---|---|---|---|---|---|---|
| C | calculé | : | 82,30 % | trouvé | : | 81,95 % |
| H | calculé | : | 9,87 % | trouvé | : | 9,94 % |

*I.R.* (film)
3100 - 3000, 1680, 1600 - 1570 $cm^{-1}$

*R.M.N.* ($CDCl_3$)
7,90 et 7,30 (2d, 4H); 3,00 (t, 2H); 2,60 (d, 2H); 2,00 - 1,50 (m, 3H); 1,00 ppm (d et t, 9H)

$n_D^{22} = 1,5095$

b) *Dichloro-2,2 (isobutyl-4 phényl)-1 propanone-1*

On fait barboter du chlore dans une solution de 190 g (1 mole) d'(isobutyl-4 phényl)-1 propanone-1 (C.P.G.: 96,9 %) dans 950 ml de N,N-diméthylformamide anhydre pendant 5,66 heures à 90 ± 5°C. Après un dégazage à l'azote, on évapore à sec sous vide. On reprend le résidu à l'eau et on extrait à l'éther éthylique.

On lave les phases organiques à l'eau, sèche et amène à sec sous vide. On obtient ainsi 248,7 g de dichloro-2,2 (isobutyl-4 phényl)-1 propanone-1 titrant 96,4 % en *C.P.G.* soit un rendement molaire de 95,4 %.

*P.E.*: 156 - 158°C sous 20 mm Hg

*Chlore*: 25,9 - 25,94% (théorie: 27,35 %) soit un titre de 94,7 %.

*I.R.* (film)
1690, 1610 $cm^{-1}$

*R.M.N.* ($CDCl_3$)
8,3 et 7,25 (2d, 4H); 2,5 (d, 2H); 2,3 (s, 3H); 1,9 (m, 1H); 0,9 ppm (d, 6H).

**Analyse**

| | | | | | | |
|---|---|---|---|---|---|---|
| C | calculé | : | 60,25 % | trouvé | : | 60,07 % |
| H | calculé | : | 6,22 % | trouvé | : | 6,09 % |
| Cl | calculé | : | 27,36 % | trouvé | : | 27,59 % |

1) En opérant comme ci-dessus à partir de l'(isobutyl-4 phényl)-1 éthanone-1, on obtient la dichloro-2,2 (isobutyl-4 phényl)-1 éthanone-1 avec un rendement molaire de 79,8 %.

On prépare un échantillon analytique par recristallisation dans le mélange hexane/toluène 1 : 2.

*P.F.*: 73°C

*C.P.G.*: 99,9 %

*Chlore*: 28,78 - 28,62 % (théorie: 28,92 %) soit un titre de 99,5 %

*I.R.*
3100 - 3000, 1690, 1600 - 1570 $cm^{-1}$

*R.M.N.* ($CDCl_3$)
8 et 7,3 (2d, 4H); 6,7 (s, 1H); 2,55 (d, 2H); 1,9 (m, 1H); 0,95 ppm (d, 6H)

**Analyse**

| | | | | | | |
|---|---|---|---|---|---|---|
| C | calculé | : | 58,79 % | trouvé | : | 59,12 % |
| H | calculé | : | 5,76 % | trouvé | : | 5,85 % |
| Cl | calculé | : | 28,92 % | trouvé | : | 28,42 % |

2) En opérant comme ci-dessus à partir de l'(isobutyl-4 phényl)-1 butanone-1, on obtient la dichloro-2,2 (isobutyl-4 phényl)-1 butanone-1 avec un rendement molaire de 97,6 %.

On prépare un échantillon analytique par distillation sous vide.

*P.E.*: 134 - 135°C sous 1,2 mm Hg

*C.P.G.*: 96,3 %

*Chlore*: 25,9 - 26,0 % (théorie: 25,95 %) soit un titre de 100 %

*I.R.*
1685, 1600 - 1570, 860 $cm^{-1}$

*R.M.N.* ($CDCl_3$)
8,20 et 7,20 (2d, 4H); 2,50 (m, 4H); 2,00 (m, 1H); 1,30 (t, 3H); 0,90 ppm (d, 6H)

**Analyse**

| | | | | | | |
|---|---|---|---|---|---|---|
| C | calculé | : | 61,55 % | trouvé | : | 61,05 % |
| H | calculé | : | 6,64 % | trouvé | : | 6,58 % |
| Cl | calculé | : | 25,95 % | trouvé | : | 25,75 % |

$n_D^{22} = 1,5305$

c) *Acide (isobutyl-4 phényl)-2 hydroxy-2 propionique*

Dans un autoclave de 500 ml, on introduit 29 (7,7.$10^{-3}$ mole) de dichloro-2,2 (isobutyl-4 phényl)-1 propanone-1 (C.P.G.: 95,4 %), 7,29 (0,18 mole) d'hydroxyde de sodium en pastilles, 200 ml d'eau et 4 ml de xylène.

On porte le mélange à 200°C et on agite pendant 1 heure à cette température.

La pression à l'intérieur de l'autoclave est de 14 bars environ.

Après avoir refroidi à 20°C, on décante et réextrait les phases aqueuses à l'éther éthylique. Après acidification à pH = 1 avec de l'acide chlorhydrique concentré, on extrait à nouveau à l'éther éthylique.

On lave à l'eau ces dernières phases éthérées, sèche sur sulfate de sodium et amène à sec sous vide.

On obtient ainsi 1,60 g d'acide (isobutyl-4 phényl)-2 hydroxy-2 propionique titrant 91,6 % en C.P.G. soit un rendement molaire de 89,8 %.

On prépare un échantillon analytique par cristallisation dans l'hexane.

C.P.G.: 99,2 %

*P.F.*: 106°C

*Titre acidimétrique*: 99,4 %

*I.R.* (KBr)
3420, 3300 - 2500, 1735 $cm^{-1}$

*R.M.N.* ($CDCl_3$)
7,6 (m, 2H); 7,4 et 7,05 (2d, 4H); 2,45 (d, 2H); 1,75 (s + m, 4H); 0,9 ppm (d, 6H).

**Analyse**

| | | | | | | |
|---|---|---|---|---|---|---|
| C | calculé | : | 70,24 % | trouvé | : | 70,63 % |
| H | calculé | : | 8,16 % | trouvé | : | 8,18 % |

En opérant comme décrit ci-dessus on a obtenu les produits suivants:

1) à partir de dichloro-2,2 (isobutyl-4 phényl)-1 éthanone-1, l'acide (isobutyl-4 phényl)-2 hydroxy-2 acétique avec un rendement molaire de 86,8 %.
On obtient un échantillon analytique par recristallisation dans l'hexane.

*P.F.*: 138°C

*C.P.G.*: 99,6 %

*Titre acidimétrique*: 99,6 %

*I.R.*
3420, 3200 - 2500, 1710 $cm^{-1}$

*R.M.N.* ($CDCl_3$)
7,15 (m, 6H); 5 (s, 1H); 2,4 (d, 2H); 1,85 (m, 1H); 0,85 ppm (d, 6H)

**Analyse**

| | | | | | | |
|---|---|---|---|---|---|---|
| C | calculé | : | 69,21 % | trouvé | : | 69,14 % |
| H | calculé | : | 7,74 % | trouvé | : | 7,76 % |

2) à partir de dichloro-2,2 (isobutyl-4 phényl)-1 butanone-1, l'acide (isobutyl-4 phényl)-2 hydroxy-2 butanoïque avec un rendement molaire de 80,9 %.
On prépare un échantillon analytique par recristallisation dans l'hexane.

*C.P.G.*: 99,3 %

*P.F.*: 117°C

*Titre acidimétrique*: 99,2 %

*I.R.*
3420, 3100 - 2700, 1720 $cm^{-1}$

*R.M.N.* ($CDCl_3$)
7,5 et 7,1 (2d + m, 6H); 2,45 (d, 2H); 2,1 (m, 3H); 0,9 ppm (t + d, 9H).

**Analyse**

| | | | | | | |
|---|---|---|---|---|---|---|
| C | calculé | : | 71,16 % | trouvé | : | 71,25 % |
| H | calculé | : | 8,53 % | trouvé | : | 8,64 % |

On a réalisé d'autres essais de préparation de l'acide (isobutyl-4 phényl)-2 hydroxy-2 propionique (A) en faisant varier les quantités d'hydroxyde de sodium, d'eau et de xylène ainsi que la température et la durée de la réaction.

| Réactifs pour 2 g de dichloro cétone | | | Réaction de transposition | | Rendements molaires (en %) | |
|---|---|---|---|---|---|---|
| NaOH (g) | eau (ml) | xylène (ml) | Température (°C) | Durée (h) | A | B |
| 7,2 | 150 | 30 | 180 | 0,25 | 77 | 7 |
| 10 | 200 | 40 | 200 | 1 | 92 | 4 |
| 7,2 | 200 | 40 | 200 | 1 | 86 | 4 |
| 7,2 | 100 | 20 | 200 | 1 | 69 | 4 |
| 7,2 | 200 | 40 | 200 | 0,25 | 90 | 4 |
| 7,2 | 120 | 20 | 200 | 0,25 | 78 | 13 |
| 7,2 | 150 | 30 | 200 | 0,25 | 84 | 8 |
| 7,2 | 200 | 40 | 200 | 0,25 | 88 | 4 |
| 3,6 | 200 | 40 | 200 | 1 | 82 | 6 |

**Exemple 2**

*Préparation de l'acide (isobutyl-4 phényl)-2 hydroxy-2 acétique*

a) *Dibromo-2,2 (isobutyl-4 phényl)-1 éthanone-1*

On ajoute en 1 heure à 40°C, une solution de 8 g (0,05 mole) de brome dans 10 ml d'acide acétique à une solution de 8,8 g (0,05 mole) d'(isobutyl-4 phényl)-1 éthanone-1 (C.P.G.: 94,5 %) dans 30 ml d'acide acétique. On agite le milieu réactionnel pendant 3 heures puis on le porte à 80°C. A cette température, on introduit en 1 heure une solution de 8 g (0,05 mole) de brome dans 10 ml d'acide acétique. On agite encore 1 heure à 80°C puis on refroidit le milieu réactionnel à 20°C. On le verse dans l'eau et on extrait à l'éther éthylique. On lave les phases organiques à l'eau, on sèche et on évapore.

On obtient ainsi 14,83 g de dibromo-2,2 (isobutyl-4 phényl)-1 éthanone-1 titrant 77,3 % en C.P.G. soit un rendement molaire de 72,6 %.

On prépare un échantillon analytique par recristallisation dans l'éther de pétrole.

*P.F.*: 68°C

*C.P.G.*: 97,7 %

*Brome*: 47,22 - 47,29 % (théorie: 47,84 %) soit un titre de 98,8 %.

*I.R.*
3100 - 3000, 1680, 1600 - 1570, 850 $cm^{-1}$

*R.M.N.* ($CDCl_3$)
7,95 et 7,25 (2d, 4H); 6,7 (s, 1H); 2,55 (d, 2H); 1,9 (m, 1H); 0,9 ppm (d, 6H).

**Analyse**

| | | | | | | |
|---|---|---|---|---|---|---|
| C | calculé | : | 43,15 % | trouvé | : | 43,02 % |
| H | calculé | : | 4,22 % | trouvé | : | 4,17 % |
| Br | calculé | : | 47,26 % | trouvé | : | 47,84 % |

En opérant comme décrit ci-dessus à partir de 1'(isobutyl-4 phényl)-1 propanone-1, on obtient la dibromo-2,2 (isobutyl-4 phényl)-1 propanone-1 sous forme d'une huile incolore avec un rendement molaire de 52,5 %.

On obtient un échantillon analytique par chromatographie sur colonne de silice, 70 - 230 mesh avec l'hexane comme éluant.

*C.P.G.*: 98,2 %

*I.R.* (film)
1675, 1600 - 1570, 860 $cm^{-1}$

*R.M.N.* ($CDCl_3$)
8,40 et 7,20 (2d, 4H); 2,75 (s, 3H); 2,60 (d, 2H); 2,20 - 1,50 (m, 1H); 0,90 ppm (d, 6H)

$n_D^{20} = 1,5665$

**Analyse**

| | | | | | | |
|---|---|---|---|---|---|---|
| C | calculé | : | 44,56 % | trouvé | : | 44,95 % |
| H | calculé | : | 4,63 % | trouvé | : | 4,64 % |
| Br | calculé | : | 45,91 % | trouvé | : | 46,74 % |

b) *Acide (isobutyl-4 phényl)-2 hydroxy-2 acétique*

En opérant comme à l'Exemple 1c mais à partir de dibromo-2,2 (isobutyl-4 phényl)-1 éthanone-1, on obtient l'acide (isobutyl-4 phényl)-2 hydroxy-2 acétique avec un rendement molaire de 93,6 %.

De même, suivant la méthode de l'Exemple 1c, on a obtenu, à partir de dibromo-2,2 (isobutyl-4 phényl)-1 propanone-1, l'acide (isobutyl-4 phényl)-2 hydroxy-2 propionique avec un rendement molaire de 89,6 %.

**Exemple 3**

*Préparation de l'acide (isobutyl-4 phényl)-2 hydroxy-2 propionique*

Dans un ballon, on introduit 1 g de dichloro-2,2 (isobutyl-4 phényl)-1 propanone-1, l'hydroxyde de métal alcalin, l'eau et 40 ml de solvant organique non polaire.

On porte le mélange à l'ébullition et on l'y maintient, sous agitation durant 24 heures.

Après avoir refroidi à 20°C, on décante et extrait les phases aqueuses à l'éther éthylique. Après acidification à pH = 1 avec de l'acide chlorhydrique concentré, on réextrait à nouveau à l'éther éthylique. On lave ces dernières phases éthérées à l'eau, on sèche sur sulfate de sodium et on amène à sec sous vide.

On obtient ainsi l'acide (isobutyl-4 phényl)-2 hydroxy-2 propionique (A) pour lequel on a enregistré les rendements suivants comparativement à l'acide isobutyl-4 benzoïque (B) en tenant compte des conditions opératoires précisées.

a) *Hydroxyde de métal alcalin*: NaOH

Conditions Rendements molaires (%)

| NaOH (g) | $H_2O$ (ml) | Solvant | A | B |
|---|---|---|---|---|
| 40 | 400 | xylène | 67 | |
| 40 | 200 | xylène | 71 | 11 |
| 20 | 400 | xylène | 85 | 2 |
| 20 | 200 | xylène | 75 | 3 |
| 10 | 200 | xylène | 74 | 3 |
| 10 | 400 | xylène | 74 | 4 |
| 40 | 400 | toluène | 61 | 1,5 |
| 40 | 400 | isobutyl benzène | 93 | 3,5 |
| 40 | 400 | naphtalène | 68 | 10,5 |
| 40 | 400 | cyclohexane | 78 | 6 |

b) *Hydroxyde de métal alcalin: KOH*

| Conditions | | | Rendements molaires (%) | |
|---|---|---|---|---|
| KOH (g) | $H_2O$ (ml) | Solvant | A | B |
| 40 | 400 | xylène | 91 | 0 |

c) *Hydroxyde de métal alcalin: LiOH*

| Conditions | | | Rendements molaires (%) |
|---|---|---|---|
| LiOH (g) | $H_2O$ (ml) | Solvant | A |
| 40 | 400 | xylène | 87 |

**Exemple 4**

*Préparation de l'acide (isobutyl-4 phényl)-2 hydroxy-2 propionique*

Dans un autoclave de 21, en acier inoxydable, équipé d'un agitateur central mécanique, on charge 180 ml de xylène industriel, 900 ml d'eau et 86,4 g (2,16 moles) d'hydroxyde de sodium en pastilles.

On ferme l'appareil et on porte la température interne à 180°C. On introduit alors, au moyen d'une pompe, 119,6 g (0,439 mole) de dichloro-2,2 (isobutyl-4 phényl)-1 propanone-1 (C.P.G: 95, 2 %) en 2 heurs, tout en maintenant l'agitation et la température à 180°C. On agite et chauffe 15 minutes supplémentaires après la fin de l'introduction puis on refroidit l'appareil dans un courant d'air.

On ajoute à la masse réactionnelle, ramenée à 20°C, 200 g de chlorure de sodium, agite 1 heure et filtre sur verre fritté. On lave le précipité par de l'acétate d'éthyle puis on le reprend dans 1080 ml d'eau. On acidifie la suspension obtenue avec 40 ml d'acide chlorhydrique concentré puis on filtre.

Après lavage à l'eau du précipité obtenu et sèchage à 50°C sous 5 mm Hg, on isole 84,54 g d'acide (isobutyl-4 phényl)-2 hydroxy-2 propionique brut, titrant 94,3 % en C.P.G. soit un rendement molaire de 81,7 %.

**Exemple 5**

*Préparation de l'acide (chloro-5 thiényl-2)-2 hydroxy-2 propionique*

a) *Dichloro-2,2 (chloro-5 thiényl-2)-1 propanone-1*

On fait barboter du chlore pendant 2h à 90 - 100°C dans une solution de 10 g ($70.10^{-3}$ mole) de propionyl-2 thiophène dans 40 ml de N,N-diméthylformamide anhydre.

Après avoir dégazé à l'azote, on évapore le N,N-diméthylformamide sous vide, puis on reprend à l'eau et on extrait à l'éther éthylique. On lave les phases organiques à l'eau salée, on les sèche sur sulfate de sodium et on les amène à sec sous vide.

On obtient ainsi 16,76 g de dichloro-2,2 (chloro-5 thiényl-2)-1 propanone-1 titrant 40,2 % en C.P.G. soit un rendement molaire de 39,5 %.

On prépare un échantillon analytique par distillation sous vide puis cristallisation dans l'hexane.

*P.F.*: 50°C

*C.P.G.*: 96,5 %

*I.R.* (KBr): 1660 $cm^{-1}$

*R.M.N.* ($CDCl_3$)
8,00 (d, 1H); 7,00 (d, 1H); 2,30 ppm (s, 3H)

**Analyse**

| | | | | | | |
|---|---|---|---|---|---|---|
| C | calculé | : | 34,52 % | trouvé | : | 34,42 % |
| H | calculé | : | 2,07 % | trouvé | : | 2,02 % |
| Cl | calculé | : | 43,68 % | trouvé | : | 43,91 % |

b) *Acide (chloro-5 thiényl-2)-2 hydroxy-2 propionique*

Dans un autoclave de 21 en acier inoxydable, équipé d'un agitateur central mécanique, on charge 120 ml d'eau, 4.32 g ($108.10^{-3}$ mole) d'hydroxyde de sodium en pastilles, 1,164 g ($4,42.10^{-3}$ mole) de dichloro-2,2 (chloro-5 thiényl-2)-1 propanone-1 et 24 ml de xylène.

On ferme l'appareil et on porte la température interne à 200°C. On agite le milieu réactionnel pendant 15 minutes à 200°C puis on refroidit appareil dans un courant d'air. On extrait à l'éther éthylique et on acidifie la phase aqueuse à pH = 1 avec de l'acide chlorhydrique concentré. On extrait à nouveau à l'éther éthylique et

on lave ces dernières phases organiques à l'eau salée. On sèche sur sulfate de sodium et on amène à sec sous vide.

On obtient ainsi 0,89 g d'acide (chloro-5 thiényl-2)-2 hydroxy-2 propionique titrant 84,3 % en C.P.G. soit un rendement molaire de 82,2 %.

On prépare un échantillon analytique par recristallisation dans le toluène.

*P.F.*: 93°C

*C.P.G.*: 95,4 %

*I.R.* (KBr)
3990, environ 2950 large, 1725 $cm^{-1}$

*R.M.N.* ($CDCl_3$)
8,20 (m, 2H); 7,00 - 6,70 (m, 2H); 1,75 ppm (s, 3H).

**Analyse**

| | | | | | | |
|---|---|---|---|---|---|---|
| C | calculé | : | 40,69 % | trouvé | : | 40,23 % |
| H | calculé | : | 3,41 % | trouvé | : | 3,43 % |
| S | calculé | : | 15,52 % | trouvé | : | 15,30 % |
| Cl | calculé | : | 17,16 % | trouvé | : | 17,61 % |

**Exemple 6**

*Préparation de l'acide hydroxy-2 phényl-2 propionique*

a) *Dichloro-2,2 phényl-1 propanone-1*

On fait barboter du chlore pendant 1,25 h à 100°C dans une solution de 33,5 g (0,25 mole) de propiophénone dans 300 ml de N,N-diméthylformamide anhydre.

On refroidit le milieu réactionnel à 20°C et on le verse dans 600 ml d'acide chlorhydrique 2 N. Après extraction à l'éther éthylique, on lave les phases organiques à l'eau, on les sèche et on les amène à sec sous vide.

On obtient ainsi 49,6 g de dichloro-2,2 phényl-1 propanone-1 possédant un titre en chlore de 96,4 % soit un rendement de 94,2 %.

Après distillation, on obtient un produit dont les caractéristiques sont les suivantes:

*I.R.* (film)
1700, 1605 $cm^{-1}$

*R.M.N.* ($CCl_4$)
8,30 et 7,45 (2m, 5H); 2,30 ppm (s, 3H)

*Chlore*: calculé: 34,9 % trouvé: 34,0 %
soit un titre de 97,4 %

b) *Acide hydroxy-2 phényl-2 propionique*

En opérant comme décrit dans l'Exemple 5 à partir de 2 g ($9,6.10^{-3}$ mole) de dichloro-2,2 phényl-1 propanone-1, on obtient 1,42 g d'acide hydroxy-2 phényl-2 propionique titrant 54,4 % en C.P.G. soit un rendement molaire de 48,5 %.

On prépare un échantillon analytique après recristallisation dans l'éther isopropylique.

*P.F.*: 84°C

*C.P.G.*: 97,6 %

*I.R.* (KBr)
3480, 3280, environ 2900 large, 1710 $cm^{-1}$

*R.M.N.* ($DMSOd_6$)
7,80 - 7,20 (m, 7H); 1,75 ppm (s, 3H).

+
+ +

Les Exemples suivants illustrent la préparation d'acides alcanoïques de formule (I') à partir d'α-hydroxy-acides de formule I.

**Exemple I**

*Préparation de l'acide (isobutyl-4 phényl)-2 propionique à partir du composé correspondant de formule I*

a) *Acide (isobutyl-4 phényl)-2 propènoïque*

On agite à reflux (avec élimination d'eau par azéotropie) pendant 2 heures, une solution de 2,22 g ($9.7.10^{-3}$ mole) d'acide (isobutyl-4 phényl)-2 hydroxy-2 propionique (C.P.G.: 96,9 %) et 2,22 g d'acide p-toluènesulfonique mono-hydraté dans 90 ml de benzène.

Après avoir refroidi à 20°C, on lave le milieu à l'eau salée, sèche sur sulfate de sodium et amène à sec sous vide.

On obtient ainsi 1,98 g d'acide (isobutyl-4 phényl)-2 propènoïque titrant 98,8 % en C.P.G. soit un rendement molaire de 99,0 %.

On prépare un échantillon analytique par recristallisation dans l'hexane.

*C.P.G.*: 100 %

*P.F.*: 95°C

*I.R.* (KBr)
3300 - 2500, 1670, 1615 - 1605, 840 $cm^{-1}$

*R.M.N.* ($CDCl_3$)
11,9 (s, 1H); 7,2 (m, 4H); 6,5 et 5,95 (2s, 2x1H); 2,45 (d, 2H); 1,8 environ (m, 1H); 0,9 ppm (d, 6H).

**Analyse**

| | | | | | | |
|---|---|---|---|---|---|---|
| C | calculé | : | 76,44 % | trouvé | : | 76,27 % |
| H | calculé | : | 7,90 % | trouvé | : | 7,88 % |

En opérant comme ci-dessus, à partir de l'acide (isobutyl-4 phényl)-2 hydroxy-2 butanoïque, on obtient l'acide (isobutyl-4 phényl)-2 butène-2 oïque avec un rendement molaire de 91,9 %.

On prépare un échantillon analytique par recristallisation dans l'hexane.

*C.P.G.*: 96,4 %

*P.F.*: 47°C

*I.R.*
Environ 3000 $cm^{-1}$, 1695 $cm^{-1}$

*R.M.N.* ($CDCl_3$)
12,3 (s, 1H); 7,3 - 6,95 (m, 4H); 6,35 (q, 1H); 2,45 (d, 2H); 2,1 (d, 2H); 1,9 (m, 1H); 0,9 ppm (d, 6H).

**Analyse**

| | | | | | | |
|---|---|---|---|---|---|---|
| C | calculé | : | 77,03 % | trouvé | : | 76,67 % |
| H | calculé | : | 8,31 % | trouvé | : | 8,27 % |

b) *Acide (isobutyl-4 phényl)-2 propionique*

On agite sous atmosphère d'hydrogène durant 1,25 heure à 20°C, une suspension de 0,80 g ($3,9.10^{-3}$ mole) d'acide (isobutyl-4 phényl)-2 propènoïque et de 40 mg de charbon palladié à 5 % de palladium, dans 16 ml d'éthanol.

Après filtration du catalyseur, on amène le milieu réactionnel à sec sous vide.

On obtient ainsi 0,80 g d'acide (isobutyl-4 phényl)-2 propionique ou ibuprofène titrant 98,1 % en C.P.G. soit

un rendement molaire de 97,1 %.

*P.F.*: 78°C

*I.R.*
Environ 3000, 1710 $cm^{-1}$

*R.M.N.* ($CDCl_3$)
9,95 (s, 1H); 7,1 (m, 4H); 3,65 (q, 1H); 2,45 (d, 2H); 1,95 (m, 1H); 1,45 (d, 3H); 0,9 ppm (d, 6H).

En opérant comme ci-dessus mais à partir de l'acide (isobutyl-4 phényl)-2 butène-2 oïque, on obtient l'acide (isobutyl-4 phényl)-2 butanoïque ou butibufène avec un rendement de 100 %.

*I.R.*
Environ 3000, 1705 $cm^{-1}$

*R.M.N.* ($CDCl_3$)
11,25 (s, 1H); 7,1 (m, 4H); 3,4 (t, 1H); 2,45 (d, 2H); 2,2 - 1,5 (m, 3H); 0,9 ppm (d + t, 9H).

**Analyse**

| | | | | | | |
|---|---|---|---|---|---|---|
| C | calculé | : | 76,33 % | trouvé | : | 76,28 % |
| H | calculé | : | 9,15 % | trouvé | : | 9,30 % |

**Exemple II**

*Préparation de l'acide (isobutyl-4 phényl)-2 propionique à partir du composé correspondant de formule I*

On agite sous atmosphère d'hydrogène pendant 29 heures à 20°C, une suspension de 2,22 g ($9,7.10^{-3}$ mole) d'acide (isobutyl-4 phényl)-2 hydroxy-2 propionique (C.P.G.: 96,9 %), 0,44 g de charbon palladié à 5 % de palladium dans 20 ml d'acide acétique et 2,2 ml d'acide sulfurique. On filtre le milieu réactionnel et on ajoute de l'eau. Après extraction à l'éther éthylique, on lave les phases organiques à l'eau, sèche sur sulfate de sodium et amène à sec sous vide.

On obtient ainsi 2,05 g d'acide (isobutyl-4 phényl)-2 propionique ou ibuprofène titrant 88 % en C.P.G. soit un rendement molaire de 90,4 %.

a) En opérant comme ci-dessus mais à partir de l'acide (isobutyl-4 phényl)-2 hydroxy-2 acétique, on obtient l'acide (isobutyl-4 phényl)-2 acétique ou ibufénac avec un rendement molaire de 95,3 %.
On prépare un échantillon analytique par recristallisation dans l'hexane.

*C.P.G.*: 99,7 %

*P.F.*: 85°C

*I.R.* (KBr)
3500 - 2500, 1695 $cm^{-1}$

*R.M.N.* ($CDCl_3$)
11,95 (s, 1H); 7,1 (m, 4H); 3,6 (s, 2H); 2,45 (d, 2H); 1,95 (m, 1H); 0,9 ppm (d, 6H)

**Analyse**

| | | | | | | |
|---|---|---|---|---|---|---|
| C | calculé | : | 74,97 % | trouvé | : | 75,38 % |
| H | calculé | : | 8,39 % | trouvé | : | 8,47 % |

b) En opérant comme ci-dessus mais à partir de l'acide (isobutyl-4 phényl)-2 hydroxy-2 butanoïque, on obtient l'acide (isobutyl-4 phényl)-2 butanoïque ou butibufène avec un rendement molaire de 83,1 %.

**Exemple III**

*Préparation de l'acide (isobutyl-4 phényl)-2 propionique à partir du composé correspondant de formule I*

On agite, durant 6 heures à 60°C, une solution de 2,22 g ($9,7.10^{-3}$ mole) d'acide (isobutyl-4 phényl)-2 hydroxy-2 propionique (C.P.G.: 96,9 %) dans 13 ml d'acide acétique et 4,4 ml d'acide iodhydrique à 57 %. On refroidit le milieu réactionnel à 20°C et on le verse dans une solution de bisulfite de sodium. Après extraction à l'éther éthylique, on lave les phases organiques à l'eau additionnée de chlorure de sodium, on sèche sur sulfate de sodium et on amène à sec sous vide.

On obtient ainsi 2,06 g d'acide (isobutyl-4 phényl)-2 propionique titrant 91 % en C.P.G., soit un rendement de 93,9 %.

## Revendications

1. Procédé de préparation d'acides α-hydroxy-alcanoïques de formule générale:

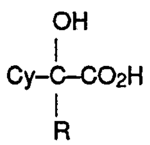

I

dans laquelle R représente hydrogène ou un radical alkyle inférieur et Cy représente un radical phényle ou hétérocyclique, ce radical comportant éventuellement un ou plusieurs substituants sélectionnés du groupe formé par des radicaux alkyle inférieur, alkényle inférieur, alkynyle inférieur et par des atomes d'halogène caractérisé en ce que:

– on traite une cétone α,α-dihalogénée de formule générale:

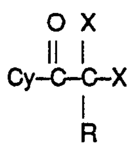

II

dans laquelle R et Cy ont la même signification que précédemment et X représente chlore, brome ou iode en présence d'une solution aqueuse d'un hydroxyde de métal alcalin et d'un solvant organique non polaire sélectionné d'un hydrocarbure aromatique ou alicyclique et ce, à une température comprise entre la température d'ébullition du milieu réactionnel à pression atmosphérique et 240°C sous pression,

– puis on acidifie le sel de métal alcalin formé pour obtenir l'acide désiré.

2. Procédé selon la Revendication 1 caractérisé en ce que l'hydroxyde de métal alcalin est l'hydroxyde de lithium, de sodium ou de potassium.

3. Procédé selon la Revendication 1 caractérisé en ce que l'hydrocarbure aromatique est un xylène.

4. Procédé selon la Revendication 1 caractérisé en ce que R représente hydrogène, méthyle ou éthyle.

5. Procédé selon la Revendication 1 caractérisé en ce que Cy représente un radical isobutyl-4 phényle.

6. Procédé selon la Revendication 1 caractérisé en ce que l'on utilise:

0,5 à 40 parties en poids d'hydroxyde de métal alcalin
5 à 400 parties en volume d'eau
2 à 40 parties en volume de solvant

et ce, pour 1 partie en poids de cétone α,α-dihalogénée, le traitement ayant lieu à la température d'ébullition du milieu réactionnel à pression atmosphérique.

7. Procédé selon la Revendication 1 caractérisé en ce que l'on utilise:

0,5 à 10 parties en poids d'hydroxyde de métal alcalin
5 à 150 parties en volume d'eau
1 à 30 parties en volume de solvant

et ce, pour 1 partie en poids de cétone α,α-dihalogénée, le traitement ayant lieu à une température comprise entre la température d'ébullition du milieu réactionnel à pression atmosphérique et 240°C sous pression.

8. Procédé selon la Revendication 1 caractérisé en ce que le traitement a lieu à une température de 180 à 220°C.

9. Procédé selon la Revendication 1 caractérisé en ce que le traitement a lieu en autoclave à une température comprise entre la température d'ébullition du milieu réactionnel à pression atmosphérique et 240°C, par introduction en continu de la cétone α,α-dihalogénée dans ledit milieu réactionnel.

10. Cétones α,α-dihalogénées de formule générale:

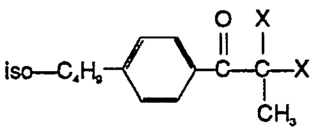

dans laquelle X représente un atome de chlore ou de brome.

**Claims**

1. A process for the preparation of α-hydroxyalkanoic acids of the general formula

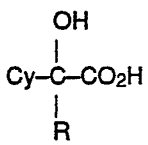

I

in which R represents hydrogen or a lower alkyl radical and Cy represents a phenyl or heterocyclic radical, this radical being unsubstituted or containing one or more substituents selected from the group comprising lower alkyl, lower alkenyl and lower alkynyl radicals and halogen atoms, characterized in that:

– an α,α-dihalogenated ketone of the general formula

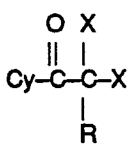

II

in which R and Cy are as defined above and X represents chlorine, bromine or iodine, is treated in the presence of an aqueous solution of an alkali metal hydroxide and a non-polar organic solvent selected from an aromatic or alicyclic hydrocarbon, said treatment being carried out at a temperature between the boiling point of the reaction medium at atmospheric pressure and 240°C under pressure, and

– the alkali metal salt formed is then acidified to give the desired acid.

2. A process according to Claim 1, characterized in that the alkali metal hydroxide is lithium, sodium or potassium hydroxide.

3. A process according to Claim 1, characterized in that the aromatic hydrocarbon is a xylene.

4. A process according to Claim 1, characterized in that R represents hydrogen, methyl or ethyl.

5. A process according to Claim 1, characterized in that Cy represents a 4-isobutylphenyl radical.

6. A process according to Claim 1, characterized in that:

0.5 to 40 parts by weight of alkali metal hydroxide,
5 to 400 parts by volume of water and
2 to 40 parts by volume of solvent

are used per 1 part by weight of α,α-dihalogenated ketone, the treatment taking place at the boiling point of the reaction

medium at atmospheric pressure.

7. A process according to Claim 1, characterized in that:

0.5 to 10 parts by weight of alkali metal hydroxide,
5 to 150 parts by volume of water and
1 to 30 parts by volume of solvent

are used per 1 part by weight of α,α-dihalogenated ketone, the treatment taking place at a temperature between the boiling point of the reaction medium at atmospheric pressure and 240°C under pressure.

8. A process according to Claim 1, characterized in that the treatment takes place at a temperature in the range from 180 to 220°C.

9. A process according to Claim 1, characterized in that the treatment takes place in an autoclave at a temperture between the boiling point of the reaction medium at atmospheric pressure and 240°C, the α,α-dihalogenated ketone being introduced continuously into said reaction medium.

10. α,α-Dihalogenated ketones of the general formula

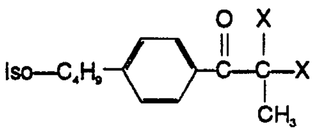

in which X represents a chlorine or bromine atom.

**Patentansprüche**

1. Verfahren zur Herstellung von α-Hydroxyalkancarbonsäuren der allgemeinen Formel (I)

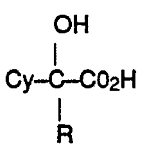

(I)

in der bedeuten: R Wasserstoff oder niedriges Alkyl und Cy Phenyl oder einen heterocyclischen Ring, die ggf. mit mindestens einem Substituenten, ausgewählt unter niedrigem Alkyl, niedrigem Alkenyl, niedrigem Alkinyl oder Halogenatomen, substituiert sind, dadurch gekennzeichnet, daß

– ein α,α-dihalogeniertes Keton der Formel (II)

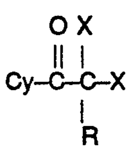

(II)

in der R und Cy die oben angegebene Bedeutung haben und X Chlor, Brom oder Jod bedeutet, in Gegenwart einer wäßrigen Lösung eines Alkalimetallhydroxyds und eines nichtpolaren organischen Lösungsmittels, ausgewählt unter einem Bromatischen oder alicyclischen Kohlenwasserstoff, bei einer Temperatur zwischen der Siedetemperatur des Reaktionsmediums bei Atmosphärendruck und 240 °C unter Druck behandelt wird und
– das gebildete Alkalimetallsalz angesäuert wird, um die gewünschte Säure herzustellen.

2. Verfahren nach Anspruch 1, gekennzeichnet durch Verwendung von Lithium-, Natrium- oder Kaliumhydroxyd als Alkalimetallhydroxyd.

3. Verfahren nach Anspruch 1, gekennzeichnet durch Verwendung von Xylol als aromatischen Kohlenwasserstoff.

4. Verfahren nach Anspruch 1, gekennzeichnet durch Verwendung einer Verbindung der Formel (II), in der R Wasserstoff, Methyl oder Ethyl ist.

5. Verfahren nach Anspruch 1, gekennzeichnet durch Verwendung einer Verbindung der Formel (II), in der Cy 4-Isobutylphenyl ist.

6. Verfahren nach Anspruch 1, gekennzeichnet durch Verwendung von

0,5 bis 40 Gewichtsteilen Alkalimetallhydroxyd,
5 bis 400 Volumenteilen Wasser und
2 bis 40 Volumenteilen Lösungsmittel,

jeweils bezogen auf 1 Gewichtsteil α,α-dihalogeniertes Keton, wobei die Behandlung bei der Siedetemperatur des Reaktionsmediums bei Atmosphärendruck durchgeführt wird.

7. Verfahren nach Anspruch 1, gekennzeichnet durch Verwendung von

0,5 bis 10 Gewichtsteilen Alkalimetallhydroxyd,
5 bis 150 Volumenteilen Wasser und
1 bis 30 Volumenteilen Lösungsmittel,

jeweils bezogen auf 1 Gewichtsteil α,α-dihalogeniertes Keton, wobei die Behandlung bei einer Temperatur zwischen der Siedetemperatur des Reaktionsmediums bei Atmosphärendruck und 240°C unter Druck durchgeführt wird.

8. Verfahren nach Anspruch 1, dadurch gekennzeichnet, daß die Behandlung bei einer Temperatur von 180 bis 220°C durchgeführt wird.

9. Verfahren nach Anspruch 1, dadurch gekennzeichnet, das die Behandlung bei einer Temperatur zwischen der Siedetemperatur des Reaktionsmediums bei Atmosphärendruck und 240°C in einem Autoklaven durch kontinuierliche Zugabe des α,α-dihalogenierten Ketons in das Reaktionsmedium durchgeführt wird.

10. α,α-dihalogenierte Ketone der allgemeinen Formel

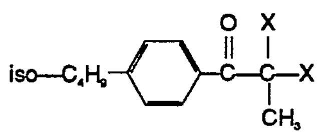

in der X Chlor oder Brom ist.